# EUROPEAN PATENT SPECIFICATION

(11) **EP 3 379 930 B1**
(45) Date of publication and mention of the grant of the patent: **30.12.2020**
(21) Application number: 16797481.5
(22) Date of filing: 07.11.2016
(51) Int. Cl.: A01N 59/16, A61Q 11/00, A61Q 19/10, A61K 8/20, A61K 33/30, C11D 3/48, C11D 9/00, C11D 9/32, C11D 10/04, A01N 31/04, A01N 31/08, A01N 43/40, A61K 8/34, A61K 8/49, A61K 31/05, A61K 31/435, A61Q 17/00, C11D 3/20

(54) **AN ANTIMICROBIAL COMPOSITION**
ANTIMIKROBIELLE ZUSAMMENSETZUNG
COMPOSITION ANTIMICROBIENNE

(30) Priority: 27.11.2015 WO PCT/EP2015/196697
(43) Date of publication of application: 03.10.2018
(73) Proprietor: Unilever N.V., 3013 AL Rotterdam (NL); Unilever PLC, London, Greater London EC4Y 0DY (GB)
(72) Inventor: AGARKHED, Ajit, Manohar, Mumbai 400 099 Chakala Andheri East (IN); GARG, Yatin, Mumbai 400 099 Chakala Andheri East (IN); HEGISHTE, Swapnil, Ravikant, Mumbai 400099 Chakala Andheri East (IN); TOMAR, Nikita, Mumbai 400 099 Chakala Andheri East (IN); WU, Guohui, Trumbull, Connecticut 06611 (US)
(74) Representative: van den Brom, Coenraad Richard
(86) International application number: PCT/EP2016/076820
(87) International publication number: WO 2017/089104

(56) References cited:
- WO-A1-2004/006876
- WO-A1-2013/064315
- US-A1- 2011 223 114
- US-A1- 2014 311 515
- US-A1- 2014 343 155
- SHINTRE M S ET AL: "Efficacy of an alcohol-based healthcare hand rub containing synergistic combination of farnesol and benzethonium chloride", INTERNATIONAL JOURNAL OF HYGIENE AND ENVIRONMENTAL HEALTH, URBAN U. FISCHER, JENA, DE, vol. 209, no. 5, 25 September 2006 (2006-09-25), pages 477-487, XP028043393, ISSN: 1438-4639, DOI: 10.1016/J.IJHEH.2006.04.006 [retrieved on 2006-09-25]

## Description

### Field of the invention

The present invention relates to an antimicrobial composition. It particularly relates to an antimicrobial composition providing sustained antimicrobial efficacy.

### Background of the invention

Human health is impacted by variety of microbes such as protozoans, bacteria, fungi, molds and viruses. For example, invasion by microbial entities including various viruses and bacteria cause a wide variety of diseases and ailments. To reduce the effects of such an invasion, people frequently wash their skin with antimicrobial soaps. Antibacterial soaps typically include soap, which is sodium or potassium salt of fatty acids, in combination with one or more antimicrobial agents like triclosan.

Insoluble particulate metal pyrithiones are acknowledged as antimicrobial agents which are usually incorporated into antimicrobial compositions, such as antidandruff hair shampoos and conditioners.

The constant threat of bacterial contamination and the associated repercussions on health have made antimicrobial solutions a ubiquitous part of commercial and residential cleaning and disinfection processes. The usual disinfecting compositions show no detectable reduction in bacterial levels on surfaces amenable to bacterial growth and proliferation in susceptible environments, such as hospitals and in residential kitchen and bath areas. On the other hand, some cleansing compositions produce substantial reduction in bacterial levels but it is generally short-lived. This often results in recontamination due to reuse of such surfaces, requiring frequent reapplication of the disinfectant. Further, relatively high concentrations of the active agent have to be incorporated in such formulations to obtain broad-spectrum disinfection. These high concentrations often have undesirable side effects such as skin and eye irritation, in addition to being potentially hazardous when in contact with food.

WO11151171 A1 (Unilever) discloses an invention in the field of skin hygiene, especially hand hygiene and/or hand soap compositions. The composition comprising lower amount of essential oil and a polymer complex or mixture provides improved hygiene efficacy.

WO2004/006876 A1 (Unilever) discloses that tulsi oil or a component compound thereof and a synthetic antimicrobial agent are capable of exhibiting a synergistic antimicrobial activity and are therefore useful in a composition for treating and/or preventing dandruff. A hair and/or scalp treatment composition comprising tulsi oil and a metal pyrithione are disclosed, wherein the tulsi oil and the metal pyrithione are capable of exhibiting synergistic antimicrobial activity. Tulsi oil comprises high amounts of eugenol. eugenol.

US 2014/0311515 describes a cleaning composition comprising a di-or tri-carboxylic acid, thymol and terpineol.

WO 2013/064315 discloses an antimicrobial cleaning composition comprising benzalkonium chloride, di-or tri-carboxylic acid, and an essential oil selected from thymol or terpineol.

US 2011/0223114 refers to an antimicrobial composition based on thymol and terpineol.

US 2014/0343155 describes an antimicrobial composition comprising thymol and terpineol and a hydrotrope selected from sodium benzoate, sodium toluene sulphonate, sodium cumene sulphonate sodium xylene sulphonate, sodium salicylate or sodium acetate, and mixtures thereof.

WO 04/035723 A1 (RECKITT BENCKISER) discloses a non-cationic antimicrobial agent containing composition which blooms when added to water. The compositions have good cleaning, disinfecting and bloom properties.

There is need for newer disinfecting compositions that provide sustained broad-spectrum microbial disinfection on surfaces over prolonged periods without reapplication, even after being contacted by cleaning solutions and after surface reuse. Furthermore, it is desirable to achieve disinfecting action using lower levels of antimicrobial agents that generally do not pose toxicity-related problems for the user. There is also a need to provide an antimicrobial cleansing composition providing sustained antimicrobial efficacy.

### Summary of the invention

According to the first aspect of the present invention, there is provided an antimicrobial composition as claimed in claim 1.

According to another aspect of the invention there is provided a method of inhibiting microbial growth on a surface, said method comprising the steps of: applying a composition of the first aspect of the invention to the surface; and rinsing the surface with a suitable solvent; wherein when said surface is animate the method is non-therapeutic.

According to yet another aspect is provided the non-therapeutic use of a composition according to the first aspect of the invention for improved personal hygiene.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples *per se.* Similarly, all percentages are weight/weight percentages unless otherwise indicated.

### Detailed description of the invention

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

The present invention provides a composition comprising polyvalent metal salt of pyrithione and benefit agent having a hydrophobicity Log-P value ranging from 1 to 6.5, more preferably 1.5 to 6 and most preferably from 1.5 to 5.5. It is preferred that polyvalent metal salt of pyrithione is Zinc pyrithione. It is further preferred that the Zinc pyrithione is present from 0.05% to 3% by weight of the composition.

The present invention provides a composition comprising polyvalent metal salt of pyrithione and antimicrobial agent having a minimum inhibitory concentration (MIC) of up to 100 ppm, more preferably up to 50 ppm and most preferably up to 20 ppm. It is preferred that polyvalent metal salt of pyrithione is Zinc pyrithione (ZPTO). It is further preferred that the Zinc pyrithione is present from 0.05% to 3% by weight of the composition.

The antimicrobial composition comprises polyvalent metal salt of pyrithione and an antimicrobial essential oil comprising thymol and terpineol.

The present invention provides an antimicrobial composition comprising: polyvalent metal salt of pyrithione; and antimicrobial essential oil comprising thymol and terpineol in the range of 0.05% to 5% by weight of the composition, such that the composition comprises 0.001 % to 3% by weight thymol, and 0.001 % to 3% by weight terpineol.

More particularly the present invention relates to an antimicrobial composition comprising polyvalent metal salt of pyrithione; 0.001 to 3% by weight thymol, and 0.001 to 3% by weight terpineol, wherein, the ratio of %weight of polyvalent metal salt of pyrithione to sum of % weight of thymol and terpineol ranges from 0.005:1 to 200:1. It is preferable that the ratio of %weight of polyvalent metal salt of pyrithione to sum of % weight of thymol and terpineol ranges from 0.05:1 to 20:1 and most preferably in the range of 0.1:1 to 10:1.

The compositions of the present invention are capable of providing antimicrobial efficacy for preferably at least up to 1 hour and more preferably for at least up to 3 hours, and most preferably for at least up to 5 hours. This time is counted from the time of application of the composition to the concerned substrate, whether animate or inanimate.

Alternatively, compositions of the present invention are capable of providing antimicrobial efficacy which may extend up to 12 hours, more preferably 18 hours and most preferably up to 24 hours.

The present inventors have surprisingly found that compositions comprising selected ingredients, namely thymol and terpineol and polyvalent metal salts of pyrithione, in selective proportions provide relatively sustained antimicrobial action.

The ingredients of the antimicrobial composition according to the invention are described below. The compositions are preferably meant for non-therapeutic use but may be used for therapeutic purpose. Compositions in accordance with this invention are useful for cleaning animate as well as inanimate surfaces. They are more particularly preferred for cleaning human body including skin, hair and oral cavity. Alternatively, the compositions are useful for cleaning hard surfaces (inanimate).

### Hydrophobicity and Log P value

Hydrophobicity is the physical property of a molecule (known as a hydrophobe) that is seemingly repelled from a mass of water.

A pure substance may distribute itself between 2 miscible solvents such as a hydrocarbon component & water. This partition coefficient is definite equilibrium physico -chemical property of a pure substance under specified conditions. It is function of the Gibbs energy of transfer from water to octanol hence describes the thermodynamic tendency of the compound to partition in different media.

Generally 1- octanol & water is the chosen to co-relate partion coefficient. The octanol -water partition coefficient of a substance X at a given temperature is represented by "P" and defined by superscripts "org" & "aq" used to denote mutually saturated phases & "oct" & 'W" for pure solvents. $P = {\left[X\right]}^{org} / {\left[X\right]}^{aq} ,$ i.e. the ratio of concentrations (mole/volume) at equilibrium : it is therefore unitless. Further, P is defined is as the quantity which is independent of concentration i.e. that value for which the solute obeys Henrys law in both solvents simultaneously. In practice P is determined at high dilution or extrapolated to zero concentration. Since P is measured over many magnitudes it is usually expressed as its decadic logarithm, log P.

Log P is the n-octanol/water partition coefficient that can be used to relate chemical structure to observed chemical behavior. Log P is related to the hydrophobic character of the molecule. The Log P values were calculated within Cerius2, using 25 QSAR+, which is a program obtained from Accelrys Inc., 9685 Scranton Road, San Diego, CA 92121. The QSAR+ descriptor A Log P and molar refractivity are calculated using the method described by Ghose Crippen (1989). In this atom-based approach, each atom of the molecule is assigned to a 30 particular class, with additive contributions to the total value of log P and molar refractivity. For more information about this descriptor, the reader is directed to Leffler and Grunwald (1963).

It is preferred that the hydrophobic compounds that are used as benefit agents in the present invention have a Log-P value ranging from 1 to 6.5, more preferably 1.5 to 6 and most preferably from 1.5 to 5.5. An example of such of the compounds is provided in the table below:

**Table 1**

| Serial. No | Compound | Log P Value |
|---|---|---|
| 1. | Limonene | 4.58 |
| 2. | Caryophylene | 5.35 |
| 3. | beta pinene | 0.94 |
| 4. | 1,8 cineole (eucalyptol) | 2.74 |
| 5. | p-cymene | 3.73 |
| 6. | Isobornyl acetate | 3.6 |
| 7. | Linalool | 2.97 |
| 8. | Terpineol | 2.69 |
| 9. | Camphor | 2.38 |
| 10. | Camphene | 4.56 |
| 11. | Alpha pinene | 4.83 |
| 12. | Terpinen-4-ol | 1.06 |
| 13. | Verbenone | 1.97 |
| 14. | Fenchone | 2.13 |
| 15. | Carvone | 2.23 |
| 16. | Terpineol | 2.67 |
| 17. | Perillyl alcohol | 3.07 |
| 18. | Limonene | 4.58 |
| 19. | Nerolidol | 5.36 |
| 20. | Farnesol | 5.31 |
| 21. | Linalool | 3.28 |
| 22. | Geraniol | 3.18 |
| 23. | Menthol | 3.2 |
| 24. | Tricholorocarbanilde | 4.798 |
| 25. | Triclosan- | 4.93 |
| 26. | 4-chloro-3,5-dimethylphenol- | 2.93 |
| 27. | Thymol- | 3.09 |
| 28. | 4-chloro-3,5-dimethylphenol- | 2.93 |

### Minimum inhibitory concentration (MIC)

Minimum inhibitory concentration (MIC) is the lowest concentration of an antimicrobial that will inhibit the visible growth of a microorganism after overnight incubation. Minimum inhibitory concentrations are important in diagnostic laboratories to confirm resistance of microorganisms to an antimicrobial agent and to monitor the activity of new antimicrobial agents. A MIC is the most basic laboratory measurement of the activity of an antimicrobial agent against an organism.

### Microdilution test

The minimum inhibitory and bactericidal concentrations (MICs and MBCs) can be determined using 96-well microtitre plates. The bacterial suspension is adjusted with sterile saline to a concentration of 1.0 × 105 cfu/mL. Compounds to be investigated are dissolved in broth LB medium (100 µL) with bacterial inoculum (1.0 × 104 cfu per well) to achieve the wanted concentrations (0.02-15.0 µL g/mL).The microplates are incubated for 24h at 28°C. The lowest concentrations without visible growth (at the binocular microscope) are defined as concentrations that completely inhibited bacterial growth (MICs). The MBCs are determined by serial sub-cultivation of 2 µL into microtitre plates containing 100 µL of broth per well and further incubation for 72h. The lowest concentration with no visible growth is defined as the MBC, indicating 99.5% killing of the original inoculum. The optical density of each well is measured at a wavelength of 655 nm by Microplate manager 4.0 (Bio-Rad Laboratories) and compared with a blank and the positive control. Streptomycin is used as a positive control using the same concentrations as in the disc diffusion test. Two replicates were done for each oil and each component.

It is preferred that the antimicrobial agent of the present invention have a minimum inhibitory concentration (MBC) of up to 100 ppm, more preferably up to 50 ppm and most preferably up to 20 ppm.

The MICs and MBCs values of some of the preferred compounds of the present invention are as follows:

| **Bacteria** | **linalyl acetate** | **linabol** | **limonene** | **α-pinene** | **β-pinene** | **1,8-cineole** | **camphor** | **carvacrol** | **thymol** | **menthol** | **streptomycin** |
|---|---|---|---|---|---|---|---|---|---|---|---|
| ***M. flavus*** | 7.0 | 4.0 | 7.0 | 5.0 | 5.0 | 4.0 | 5.0 | 0.02 | 0.25 | 0.5 | 1.0 |
| | 8.0 | 4.0 | 7.0 | 5.0 | 5.5 | 5.0 | 6.0 | 0.05 | 0.5 | 1.0 | 1.5 |
| ***B. subtilis*** | 7.0 | 4.0 | 7.0 | 5.0 | 5.0 | 4.0 | 5.5 | 0.125 | 0.25 | 0.5 | 1.0 |
| | 8.0 | 4.0 | 7.0 | 6.0 | 6.0 | 5.0 | 6.0 | 0.25 | 0.5 | 1.0 | 1.5 |
| ***S. epidermidis*** | 8.0 | 4.0 | 8.0 | 6.0 | 6.0 | 4.0 | 6.0 | 0.25 | 0.25 | 1.0 | 1.0 |
| | 9.0 | 5.0 | 3.0 | 6.0 | 6.5 | 5.0 | 6.0 | 0.25 | 0.5 | 1.0 | 1.5 |
| ***S. aureus*** | 8.0 | 5.0 | 8.0 | 6.0 | 6.0 | 5.0 | 6.0 | 0.25 | 0.25 | 1.0 | 1.0 |
| | 9.0 | 5.0 | 8.0 | 7.0 | 7.5 | 6.0 | 6.5 | 0.5 | 0.5 | 1.0 | 1.5 |
| ***S. enteritidis*** | 9.0 | 5.0 | 9.0 | 8.0 | 9.0 | 5.0 | 6.0 | 0.5 | 0.5 | 1.0 | 1.5 |
| | 10.0 | 6.0 | 10.0 | 9.0 | 9.0 | 6.0 | 7.0 | 0.5 | 1.0 | 1.5 | 2.0 |
| ***S. typhimurium*** | 9.0 | 5.0 | 9.0 | 8.0 | 8.0 | 5.0 | 6.0 | 0.5 | 0.5 | 1.0 | 1.5 |
| | 10.0 | 6.0 | 10.0 | 9.0 | 9.0 | 6.0 | 7.0 | 0.5 | 1.0 | 1.5 | 2.0 |
| ***E. coli*** | 10.0 | 6.0 | 10.0 | 8.0 | 8.0 | 6.0 | 7.0 | 0.5 | 1.0 | 1.0 | 2.0 |
| | 12.0 | 7.0 | 12.0 | 10.0 | 10.0 | 8.0 | 8.0 | 0.5 | 1.5 | 2.0 | 3.0 |
| ***E. cloacae*** | 10.0 | 6.0 | 10.0 | 8.0 | 9.0 | 6.0 | 7.0 | 0.5 | 1.0 | 2.0 | 2.0 |
| | 12.0 | 7.0 | 10.0 | 10.0 | 10.0 | 8.0 | 9.0 | 0.5 | 1.5 | 2.0 | 4.0 |
| ***P. mirabilis*** | 10.0 | 6.0 | 10.0 | 8.0 | 9.0 | 6.0 | 7.0 | 0.5 | 1.0 | 2.0 | 3.0 |
| | 15.0 | 8.0 | 15.0 | 10.0 | 10.0 | 8.0 | 9.0 | 1.0 | 1.5 | 3.0 | 4.0 |
| ***P. aeruginosa*** | 10.0 | 7.0 | 10.0 | 10.0 | 10.0 | 7.0 | 7.0 | 0.5 | 1.0 | 3.0 | 3.0 |
| | 15.0 | 9.0 | 15.0 | 12.0 | 13.0 | 9.0 | 10.0 | 1.0 | 1.5 | 4.0 | 5.0 |
| ***L. monocytogenes*** | 9.0 | 5.0 | 8.0 | 8.0 | 9.0 | 5.0 | 7.0 | 0.5 | 1.0 | 2.0 | 2.0 |
| | 10.0 | 6.0 | 10.0 | 10.0 | 10.0 | 6.0 | 7.0 | 0.5 | 1.0 | 2.0 | 3.0 |

### Thymol

The antimicrobial compositions of the invention comprise 0.001 to 3 %, more preferably 0.01 to 1%, and most preferably 0.05 to 0.6% by weight thymol. Most of the useful antimicrobial compositions of the present invention have thymol higher than 0.05 but lesser than 0.8% by weight. These ranges are preferred because below the preferred lower concentration of thymol, the desired faster acting antimicrobial kinetics may not be met under all circumstances. At concentrations higher than the higher range, while the kinetics of action would not be compromised in combination with terpineol,, the sensorial aspects like smell and skin feel could be compromised. Thymol may be added to the antimicrobial composition in purified form.

Alternatively, a suitable amount of thyme oil or thyme extract comprising the desired amount of thymol may be included. Thyme oil or thyme extract is obtained from the thyme plant, belonging be genus *Thymus* and includes but is not limited to the following species: *Thymus vulgaris*, *Thymus zygis, Thymus satureoides, Thymus mastichina, Thymus broussonetti, Thymus maroccanus*, *Thymus pallidus, Thymus algeriensis, Thymus serpyllum, Thymus pulegoide,* and *Thymus citriodorus.*

The structures of thymol and its isomer carvacrol are given below:

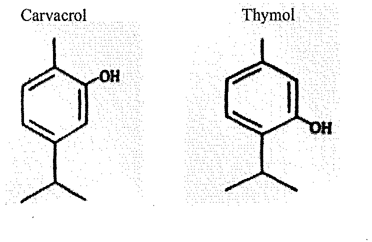

### Terpineol

The antimicrobial compositions of the invention comprise 0.001 to 3%, more preferably 0.05 to 1%, and most preferably 0.1 to 0.8% terpineol by weight of the composition of the present invention. Most of the useful fast acting antimicrobial compositions of the present invention have terpineol higher than 0.05 but lesser than 1% by weight terpineol. These preferred concentrations ranges of terpineol are important for same reasons indicated in the context of thymol. The terpineol is preferably selected from alpha-terpineol, beta-terpineol, gamma-terpineol or mixtures thereof. It is particularly preferred that the terpineol is alpha-terpineol. Terpineol may be added to the antimicrobial composition in purified form.

Alternatively, an amount of pine oil comprising desired amount of terpineol in it may be included in the antimicrobial composition. The structure of a terpineol compound is given below:

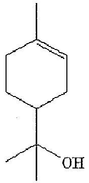

It is preferred that the sum of percentages of Thymol and Terpineol by weight is from 0.01 to 3%, more preferably from 0.1 to 2% and most preferably from 0.1 to 1% by weight of the composition.

It is particularly preferred that antimicrobial compositions of the invention comprise thymol and terpineol at w/w ratio of 1:1.

### Polyvalent metal salt of pyrithione

The insoluble metal pyrithione may be represented by the following general formula: 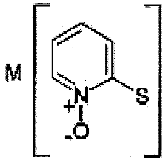 in which M is a polyvalent metal ion and n corresponds to the valency of M.

Preferred examples of M include magnesium, barium, strontium, zinc, cadmium, tin and zirconium. Especially preferred is zinc.

The metal pyrithione may have any particle form suitable for use in a composition for topical application to the skin. For example, the metal pyrithione may be in the form of amorphous or crystalline particles having a range of different particle sizes.

The metal pyrithione may, for example, be in the form of particles having size distribution in which at least about 90% of the particles have a size of up to 100 µm, more preferably up to 50 µm, even more preferably up to 10 µm, most preferably 5 µm or less, for example the size distribution may be such that at least about 90% of the particles have a size of 1 µm or less. In particular for hair compositions, smaller sizes are optimal for antimicrobial effect.

Various methods for producing fine particles of metal pyrithione are described, for example, in EP0173259 B1 (Kao Corp, 1991). Suitable methods for determining particle size are described in that publication.

The insoluble metal pyrithione may be made up of one particulate form or two or more different particulate forms.

Other suitable particulate forms for the metal pyrithione include platelets and needle-shaped particles. Platelets of zinc pyrithione are described in EP0034385 B1 (P&G, 1984). The needle-shaped particles are preferably of the type described in WO99/66886 A1 (Unilever), the contents of which are incorporated herein by reference. For needle-shaped particles preferably at least 50% of the particles are needle-shaped particles having a length of between 1 µm and 50 µm.

A preferred amount of pyrithione for the composition of the present invention is from about 0.001 % to about 3% by weight of the total composition, more preferably from about 0.05% to about 3% by weight, most preferably between 0.1% and 1% by weight.

### Additional ingredients

### Surfactant

Antimicrobial compositions of the present invention further comprise a surfactant. The antimicrobial composition of the invention is useful in personal cleansing applications. This could be a single surfactant but usually it is a combination of various types of surfactants aggregating to the claimed percentage.

In applications meant for personal cleansing, it is preferred that the surfactant is a nonionic surfactant, such as C₈-C₂₂, preferably C₈-C₁₆ fatty alcohol ethoxylates, comprising between 1 and 8 ethylene oxide groups, especially when the product is in the liquid form.

Alternatively, the surfactant is a non-soap anionic surfactant. The term non-soap surfactant is well known in the art and is used to distinguish the anionic surfactants based on their origin/composition.

The non-soap surfactants are preferably selected from primary alkyl sulphate, secondary alkyl sulphonates, alkyl benzene sulphonates, or ethoxylated alkyl sulphates. Suitable examples include alkyl ether sulphate preferably those having between 1 and 3 ethylene oxide groups,. Alkyl polyglucoside may also be present in the composition, preferably those having a carbon chain length between C6 and C16.

Thus, in a highly preferred aspect, the antimicrobial compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90 % by weight of the composition. In general, the surfactants may be chosen from the surfactants described in well-known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

When surfactant is used, a particularly preferred surfactant is soap. Soap is a surfactant suitable for human hygiene. When the anionic surfactant is a soap it is preferred that the soap is C₈-C₂₄ soap, more preferably C₁₀-C₂₀ soap and most preferably C₁₂-C₁₈ soap. The soap may or may not have unsaturation. The cations in the soap molecules can be alkali metal, alkaline earth metal or ammonium ions. Preferably, the cation is sodium, potassium or ammonium. More preferably the cation is sodium or potassium.

The soap is usually obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soya bean and castor. The fatty acid soaps can also be synthetically prepared (e.g. by the oxidation of petroleum or by the hydrogenation of carbon monoxide by the Fischer-Tropsch process). Resin acids, such as those present in tall oil, may be used. Naphthenic acids are also suitable.

Tallow fatty acids can be derived from various animal sources and generally comprise about 1 to 8% myristic acid, about 21 to 32% palmitic acid, about 14 to 31% stearic acid, about 0 to 4% palmitoleic acid, about 36 to 50% oleic acid and about 0 to 5% linoleic acid. A typical distribution is 2.5% myristic acid, 29% palmitic acid, 23% stearic acid, 2% palmitoleic acid, 41.5% oleic acid, and 3% linoleic acid by weight of soap. Other similar mixtures, such as those from palm oil and those derived from various animal tallow and lard are also included.

Coconut oil refers to fatty acid mixtures having an approximate carbon chain length distribution of 8% C₈, 7% C₁₀, 48% C₁₂, 17% C₁₄, 8% C₁₆, 2% C₁₈, 7% oleic and 2% linoleic acids (the first six fatty acids listed being saturated) by weight of soap. Other sources having similar carbon chain length distributions, such as palm kernel oil and babassu kernel oil, are included within the term coconut oil.

A typical fatty acid blend consisted of 5 to 30% coconut fatty acids and 70 to 95% fatty acids by weight of soap. Fatty acids derived from other suitable oils/fats such as groundnut, soybean, tallow, palm and palm kernel may also be used in other desired proportions.

It is preferred that compositions in accordance with the invention comprise 10 to 85% by weight soap, more preferably 25 to 75% by weight of the composition.

Preferred compositions may include other known ingredients such as perfumes, pigments, preservatives, emollients, sunscreens, emulsifiers, gelling agents and thickening agents. Choice of these ingredients will largely depend on the format of the composition.

### Carrier

The antimicrobial composition may be in form of a solid, a liquid, a gel or a paste. A person skilled in the art can prepare compositions in various formats by choosing one or more carrier materials and/or surfactant. The antimicrobial compositions of the present invention are useful for cleansing and care, in particular for skin cleansing and skin care. It is envisaged that the antimicrobial composition can be used as a leave-on product or a wash-off product, preferably a wash-off product. The antimicrobial composition of the present invention can also be used for cleansing and care of hard surfaces such as glass, metal, plastic and the like.

According to one aspect water is a preferred carrier. When water is present, it is preferably present in at least 1%, more preferably at least 2%, further more preferably at least 5% by weight of the composition. When water is the carrier, a preferred liquid composition comprises 0.05 to 3% by weight thymol, 0.05 to 3% by weight terpineol, 0.001 % to 3% by weight polyvalent metal salt of pyrithione and 10 to 99.9% by weight water.

The liquid antimicrobial composition is useful as a skin antiseptic liquid, for skin cleansing, in particular for hand wash or a face wash.

When water is the carrier, another preferred solid composition comprises 0.05 to 3% by weight thymol, 0.05 to 3% by weight terpineol, 0.001% to 3% by weight polyvalent metal salt of pyrithione, and 5 to 30% by weight water.

The solid antimicrobial composition is preferably in form of a shaped solid, more preferably a bar of soap. The solid antimicrobial composition is particularly useful for skin cleansing in particular for bathing or hand wash or face wash.

According to another aspect, inorganic particulate material is also a suitable carrier. When inorganic particulate material is the carrier, the antimicrobial composition is in a solid form. Preferably the inorganic particulate material is talc. When the inorganic particulate material is talc, the solid antimicrobial composition is particularly useful as a talcum powder for application on face or body.

According to a further aspect, a solvent is a preferred carrier. Although any solvent can be used, alcohol is a preferred solvent. Short chain alcohols, in particular ethanol and propanol, are particularly preferred as carrier for an antimicrobial wipe or an antimicrobial hand sanitizer composition.

In another aspect of the present invention, the composition of the present invention is suitable for use in wipes for personal hygiene or surface cleaning.

According to another aspect of the present invention there is provided a method of disinfecting a surface comprising the steps of applying a composition of the first aspect of the invention on to the surface; and rinsing the surface with a suitable solvent.

The solvent for rinsing the surface is preferably water but could also be a mixture of water and alcohol. The word rinsing herein includes the act of wiping the surface with a suitable wipe. Thus the surface e.g. hand, face, body, oral cavity or any hard surface e.g. a utensil is first contacted with the composition of the invention. It is then rinsed preferably with sufficient amounts of water after a pre-determined period of time to remove any visible or sensory reside of the composition. Alternatively, an alcohol wipe or a water/alcohol impregnated wipe may be used to wipe the surface to be visibly free of the anti-microbial composition. The step of rinsing the substrate is preferably carried out less than 5 minutes, preferably less than 2 minutes, further more preferably less than a minute and in many cases less than 15 seconds after the step of applying the composition on the substrate.

According to one aspect, the invention provides for non-therapeutic benefits.

Thus, according to yet another aspect of the invention there is provided use of a composition of the present invention for faster reduction in microbial count.

According to yet another aspect of the invention there is provided use of a composition comprising 0.01 to 3% by weight thymol, 0.01 to 3% by weight terpineol, and 0.001% to 3% by weight polyvalent metal salt of pyrithione and a carrier for improved personal hygiene, preferably of surfaces of human body, more preferably the human surfaces include skin, hands and oral cavity.

A preferred aspect provides for use of a composition comprising 0.01 to 2.5% by weight thymol, 0.01 to 3 % by weight terpineol, 0.001% to 3% by weight polyvalent metal salt of pyrithione and a carrier for improved hand hygiene.

The invention also provides for therapeutic benefits.

The composition of the present invention is suitable for use as an anti-dandruff treatment.

The inventors have determined that while a combination of thymol and terpineol alone do provide the fast antimicrobial kinetic action, a combination of thymol and terpineol along with the polyvalent metal salt of pyrithione provide synergistic sustained antimicrobial action, which is especially important in a wash-off product (as opposed to a leave-on product) where the contact time of the antimicrobial actives with the surface is low. The present composition provides for improvement in extent of reduction in microbial counts on the surface for a sustained period of time when surface is contacted with a composition of the present invention and rinsed off.

The surface is an animate surface such as human body or any part thereof. Alternatively, the surface is an inanimate surface such as tabletops and tiles. When the surface is animate, the method is non-therapeutic. Such methods include cosmetic methods.

Alternatively, the method is therapeutic. Treatment by therapy is defined as any treatment which is designed to cure, alleviate, remove or lessen the symptoms of, or prevent or reduce the possibility of contracting any disorder or malfunction of human or animal body.

In accordance with another aspect is disclosed use as claimed in claim 12 wherein said use is non-therapeutic in nature.

In accordance with another aspect is disclosed an antimicrobial composition of the first aspect for use to therapeutically inhibit microbial growth on an animate surface e.g. human body.

### Examples

The invention will now be demonstrated with examples. The examples are for the purpose of illustration only and they do not limit the scope of claims in any manner. The examples were conducted by adding various actives on the following base composition of soap bars:

**Table 1: Base Composition of the soap bars**

| Ingredients | % by weight |
|---|---|
| Anhydrous Soap | 72.0 |
| Glycerine | 4.0 |
| Primary Alkyl Sulphate (PAS) | 2.0 |
| Sodium Chloride | 0.7 |
| Talc | 8.0 |
| Water and other minor ingredients made up to 100% | - |
| Total | 100.0 |

### Example 1: Antimicrobial efficacy of the composition

### Preparation of bacteria (E.coli Culture)

*Escherichia coli* ATCC 10536 was obtained as a lyophilized culture from American Type Culture Collection. The test culture was grown for 24 h on Tryptic Soy Agar (TSA) streak plate at 37.0 °C. Then *E.coli* suspension was prepared at 1∼5X10⁷ CFU/ml with Tryptone Sodium Chloride right before the efficacy tests.

### Sustained Antimicrobial Efficacy Test

To determine efficacy of an antimicrobial soap bar, in-vitro performance tests were performed on artificial skin samples (VITRO-SKIN™, IMS Corp., a synthetic substrate designed to mimic the surface chemistry of human skin). To prepare the substrate, pieces of VITRO-SKIN were hydrated overnight in a hydration chamber with a reservoir of 85% water and 15% glycerin. After approximately 24 hours, the VITRO-SKIN pieces were taken out of the chamber and kept under ambient temperature and humidity for approximately one hour. Five cm diameter circular sections were mounted between the opposing pieces of an XRF cup.

To mimic washing the skin, the soap bar was cut into a 1 cm diameter cylinder and wetted deionised water, the bar soap composition gently rubbed across the entire VITRO-SKIN surface inside the XRF cup for 15 seconds. Then, the lather was generated by continuously rubbing the VITRO-SKIN with a Teflon rod for 45 seconds (e.g. absent the bar soap composition). The wash liquor was removed and the VITRO-SKIN was rinsed by adding 4ml of deionized water to the XRF cup, and the substrate was rubbed with a clean Teflon rod for 30 seconds. The rinse step was repeated one more time. After removing the rinse liquor, the VITRO-SKIN was allowed to dry for 5 to 10 minutes in still room air under low light conditions.

Each VITRO-SKIN™ was inoculated evenly with 10⁶ to 10⁷ CFUs *E.coli* by using 200 µl of culture obtained from an overnight growth as described above. The bacteria were allowed to dry on the VITRO-SKIN for 20 minutes, then the VITRO-SKIN was placed inside an incubator for 0 to 3 hours. After incubation, 10 ml ice cold D/E broth was added into each XRF cup, which was covered with Teflon caps tightly and was vigorously shaken for 1 minute to dislodge the bacteria. Serial dilutions of the fluids were made and were plated for colony count with Tryptic Soy Broth for 24 hours at 37 °C. Thereafter, the viable bacteria were counted and reported in the form of log₁₀ CFU. The smaller log₁₀ (CFU/ml) value correspond to more potent and sustained antimicrobial efficacy of a given sample.

**Table 2: Examples of antimicrobial efficacy**

| Details of the composition(s) | Log (Viable *E*. *coli*) | |
|---|---|---|
| | 0 time | 3 hour |
| 100 % by weight Base Composition only (No zinc pyrithone, No thymol, No terpineol) | 7.0 | 7.0 |
| 99.65 % by weight Base Composition + 0.35 % by weight TT which is (0.175 % by weight thymol + 0.175 % by weight terpineol) | 7.0 | 7.0 |
| 99.65 % by weight Base Composition + 0.35 % by weight ZPTO (zinc pyrithione) | 7.0 | 6.8 |
| 99.30 % by weight Base Composition + 0.35 % by weight ZPTO + 0.35 % by weight TT which is (0.175 % by weight thymol + 0.175 % by weight terpineol) | 7.0 | 6.0 |

| | | |
|---|---|---|
| Note: in the above table(s), the term TT is used to collectively represent Thymol and Terpineol | | |

The data in Table 2 show that the composition of the present invention provide longer lasting antimicrobial benefits for up to 3 hours. The data further shows that the surface is disinfected when it is treated with a composition in accordance with the invention (row-4). In row 4, the ratio (w/w) of ZPTO to the sum of percentage weight of thymol and terpineol (which cumulatively is 0.35 wt%) is 1:1.

## Claims

1. An antimicrobial composition comprising:
(a) polyvalent metal salt of pyrithione;
(b) 0.001% to 3% by weight thymol, and
(c) 0.001 % to 3% by weight terpineol based on total weight of the composition;
wherein, the ratio of %weight of polyvalent metal salt of pyrithione to sum of % weight of thymol and terpineol ranges from 0.005:1 to 200:1.

2. An antimicrobial composition as claimed in claim 1, wherein the polyvalent metal salt of pyrithione is 0.001% to 3% by weight of the composition.

3. An antimicrobial composition as claimed in claim 1 or 2, wherein the polyvalent metal salt of pyrithione zinc pyrithione.

4. An antimicrobial composition as claimed in any of the preceding claims, wherein the antimicrobial composition further comprises a surfactant.

5. An antimicrobial composition as claimed in claim 4, wherein the surfactant comprises soap.

6. An antimicrobial composition as claimed in any of claims 1 to 5 wherein the terpineol is selected from the group of alpha-terpineol, beta-terpineol, gamma-terpineol and combinations thereof.

7. An antimicrobial composition as claimed in claim 6, wherein the terpineol comprises alpha-terpineol.

8. An antimicrobial composition as claimed in any of claims 1 to 7 wherein said composition comprises thymol and terpineol at w/w ratio of 1:1.

9. A method of inhibiting microbial growth on a surface comprising the steps of
(a) applying a composition as claimed in any one of the preceding claims on to the surface; and,
(b) rinsing the surface with a suitable solvent;
wherein when said surface is animate the method is non-therapeutic.

10. A method as claimed in claim 9 wherein said surface is animate or inanimate.

11. Non-therapeutic use of a composition according to any of claims 1 to 8 for improved personal hygiene.

12. An antimicrobial composition as claimed in any of claims 1 to 8 for use to therapeutically inhibit microbial growth on an animate surface.

## Patentansprüche

1. Antimikrobielle Zusammensetzung, umfassend:
(a) mehrwertiges Metallsalz von Pyrithion;
(b) 0,001 bis 3 Gewichts-% Thymol und
(c) 0,001 bis 3 Gewichts-% Terpineol, bezogen auf das Gesamtgewicht der Zusammensetzung;
wobei das Verhältnis von Gewichtsprozent des mehrwertigen Metallsalzes von Pyrithion zur Summe von Gewichtsprozent von Thymol und Terpineol im Bereich von 0,005:1 bis 200:1 liegt.

2. Antimikrobielle Zusammensetzung wie in Anspruch 1 beansprucht, wobei das mehrwertige Metallsalz von Pyrithion 0,001 bis 3 Gewichts-% der Zusammensetzung beträgt.

3. Antimikrobielle Zusammensetzung wie Anspruch 1 oder 2 beansprucht, wobei das mehrwertige Metallsalz von Pyrithion Zinkpyrithion ist.

4. Antimikrobielle Zusammensetzung wie in irgendeinem der vorhergehenden Ansprüche beansprucht, wobei die antimikrobielle Zusammensetzung ferner ein Tensid enthält.

5. Antimikrobielle Zusammensetzung wie in Anspruch 4 beansprucht, wobei das Tensid Seife umfasst.

6. Antimikrobielle Zusammensetzung wie in irgendeinem der Ansprüche 1 bis 5 beansprucht, worin das Terpineol ausgewählt ist aus der Gruppe von alpha-Terpineol, beta-Terpineol, gamma-Terpineol und Kombinationen davon.

7. Antimikrobielle Zusammensetzung wie Anspruch 6 beansprucht, wobei das Terpineol alpha-Terpineol umfasst.

8. Antimikrobielle Zusammensetzung wie in irgendeinem der Ansprüche 1 bis 7 beansprucht, wobei die Zusammensetzung Thymol und Terpineol in einem Gew./Gew.-Verhältnis von 1:1 umfasst.

9. Verfahren zur Hemmung von mikrobiellem Wachstum auf einer Oberfläche, umfassend die folgenden Schritte
(a) Aufbringen einer Zusammensetzung wie in irgendeinem der vorhergehenden Ansprüche beansprucht auf die Oberfläche; und
(b) Spülen der Oberfläche mit einem geeigneten Lösungsmittel;
wobei das Verfahren nichttherapeutisch ist, wenn die Oberfläche belebt ist.

10. Verfahren wie in Anspruch 9 beansprucht, bei dem die Oberfläche belebt oder unbelebt ist.

11. Nichttherapeutische Verwendung einer Zusammensetzung nach irgendeinem der Ansprüche 1 bis 8 zur Verbesserung der Körperhygiene.

12. Antimikrobielle Zusammensetzung wie in irgendeinem der Ansprüche 1 bis 8 beansprucht zur Verwendung zur therapeutischen Hemmung von mikrobiellem Wachstum auf einer belebten Oberfläche.

## Revendications

1. Composition antimicrobienne comprenant :
(a) un sel de métal polyvalent de pyrithione ;
(b) 0,001 % à 3 % en masse de thymol, et
(c) 0,001 % à 3 % en masse de terpinéol sur la base de la masse totale de la composition ;
dans laquelle, le rapport de % en masse de sel de métal polyvalent de pyrithione à la somme de % en masse de thymol et terpinéol est de 0,005:1 à 200:1.

2. Composition antimicrobienne selon la revendication 1, dans laquelle le sel de métal polyvalent de pyrithione est de 0,001 % à 3 % en masse de la composition.

3. Composition antimicrobienne selon la revendication 1 ou 2, dans laquelle le sel de métal polyvalent de pyrithione est la pyrithione de zinc.

4. Composition antimicrobienne selon l'une quelconque des revendications précédentes, dans laquelle la composition antimicrobienne comprend de plus un tensioactif.

5. Composition antimicrobienne selon la revendication 4, dans laquelle le tensioactif comprend du savon.

6. Composition antimicrobienne selon l'une quelconque des revendications 1 à 5, dans laquelle le terpinéol est choisi dans le groupe d'alpha-terpinéol, béta-terpinéol, gamma-terpinéol et combinaisons de ceux-ci.

7. Composition antimicrobienne selon la revendication 6, dans laquelle le terpinéol comprend de l'alpha-terpinéol.

8. Composition antimicrobienne selon l'une quelconque des revendications 1 à 7, dans laquelle ladite composition comprend du thymol et terpinéol à un rapport masse/masse de 1:1.

9. Procédé d'inhibition de croissance microbienne sur une surface comprenant les étapes de
(a) application d'une composition selon l'une quelconque des revendications précédentes sur la surface ; et,
(b) rinçage de la surface avec un solvant approprié ;
dans lequel lorsque ladite surface est vivante le procédé est non-thérapeutique.

10. Procédé selon la revendication 9, dans lequel ladite surface est vivante ou non vivante.

11. Utilisation non-thérapeutique d'une composition selon l'une quelconque des revendications 1 à 8 pour une hygiène de la personne améliorée.

12. Composition antimicrobienne selon l'une quelconque des revendications 1 à 8 pour une utilisation pour inhiber thérapeutiquement une croissance microbienne sur une surface vivante.
